# FASCICULE DE BREVET EUROPEEN

(11) **EP 2 552 294 B1**
(45) Date de publication et mention de la délivrance du brevet: **14.02.2018**
(21) Numéro de dépôt: 11717339.3
(22) Date de dépôt: 27.03.2011
(51) Int. Cl.: A61B 1/24, A61C 5/90

(54) **DISPOSITIF D'ACCESSIBILITE BUCCALE**
VORRICHTUNG ZUM ZUGRIFF AUF DEN MUNDRAUM
ORAL ACCESSIBILITY DEVICE

(30) Priorité: 29.03.2010 FR 1052303
(43) Date de publication de la demande: 06.02.2013
(73) Titulaire: Centre Hospitalier Regional Metz Thionville, 57000 Metz (FR)
(72) Inventeur: ANASTASIO, Daniel, F-57100 Thionville (FR)
(74) Mandataire: Brungard, Yves Francois
(86) Numéro de dépôt international: PCT/IB2011/051288
(87) Numéro de publication internationale: WO 2011/121513

(56) Documents cités:
- WO-A1-2009/134295
- CN-Y- 201 098 154
- DE-A1- 19 808 419
- GB-A- 739 546
- GB-A- 2 421 678
- US-A- 4 919 616
- US-A- 4 997 368
- US-B1- 7 066 735

## Description

La présente invention se situe dans le domaine des dispositifs utilisés notamment lors d'un examen bucco-dentaire, pour faciliter l'accessibilité de la cavité buccale au praticien ou à son assistant.

Elle concerne plus particulièrement un dispositif d'accessibilité buccale présentant des fonctionnalités voisines de celles des ouvre-bouches ou similaires déjà connus, mais particulièrement adapté pour des personnes en situation de handicap comportemental.

Les ouvre-bouches connus visent essentiellement à faciliter l'accès de la cavité buccale en maintenant la bouche du patient suffisamment ouverte. Ainsi, les cale-bouches, sous forme de blocs par exemple en caoutchouc, sont des dispositifs de maintien de la bouche en position ouverte, mis en place entre les arcades dentaires après que la bouche du patient a été suffisamment ouverte. Ces dispositifs permettent de maintenir l'écartement des arcades dentaires, sans que le patient ait besoin de maintenir un effort d'ouverture buccale qui peut durer relativement longtemps, et accessoirement permettent aussi de maintenir la langue ou bien sont combinés avec des moyens d'aspiration.

Différents types de cale-bouches sous forme de blocs à insérer entre les arcades dentaires sont déjà connus, par exemple par US4997368, US7066735, WO03037205, US6244866. On connaît aussi d'autres dispositifs de type cale bouche, réalisés par exemple sous forme d'arceaux métalliques (US4053984) ou d'un simple cylindre (US2008038689), tous ces dispositifs visant à maintenir la bouche ouverte mais ne pouvant être mis en place qu'après son ouverture. Certains systèmes, tel que montré par exemple dans WO2009134295, combinent en un même dispositif un cale-bouche et des moyens d'aspiration.

On connaît aussi les ouvre-bouches articulés, à écartement fixe ou réglable, tels que par exemple décrit dans US5097820 ou WO2007137295, ou encore à crémaillère comme décrit dans WO2006054301. Ces dispositifs peuvent généralement être mis en place bouche fermée et utilisés pour écarter les arcades dentaires en exerçant un effort d'écartement subi par le patient, et ensuite à maintenir l'ouverture de la bouche. Ils peuvent présenter l'avantage d'une plus grande possibilité de réglage de l'écartement, ou de positionnement dans la bouche. Ces dispositifs sont notamment destinés à être mis en place sous anesthésie, lorsque le patient ne peut pas ouvrir lui-même la bouche volontairement. Dans d'autres cas, ils peuvent être traumatiques car ils sont réalisés en métal et susceptibles de provoquer des fractures dentaires si le patient vient à vouloir serrer fortement les arcades dentaires.

Il existe aussi des dispositifs d'ouvre bouche en forme de toupie en bois ou matière plastique, présentant une forme conique avec une sorte de filet hélicoïdal réalisé sur la surface conique. Ces dispositifs peuvent être insérés entre les arcades dentaires pratiquement serrées, et la rotation de la toupie la fait avancer entre les dents, grâce au filetage, en provoquant un écartement du fait de la conicité. Ils agissent ensuite à la manière des cale-bouches pour maintenir l'écartement des arcades dentaires.

Chez les personnes en situation de handicap comportemental, l'accessibilité de la cavité buccale est parfois limitée voire impossible. Ces patients expriment des réflexes instinctifs de protection et de défense en limitant spontanément l'amplitude de l'ouverture buccale ou en ne maintenant cette dernière que durant des périodes très courtes, ou encore en cherchant à enlever un dispositif qui les gène. Face à cette problématique, la réalisation des examens bucco-dentaires de prévention ou les soins apportés à ces patients sont difficiles.

En effet, les dispositifs de type cale-bouche ne peuvent être mis en place si la bouche n'est pas ouverte préalablement, et de plus, étant des dispositifs passifs maintenus uniquement par l'effort de serrage entre les arcades dentaires, ils risquent de ne pas rester en place si le patient écarte lui-même les arcades dentaires de manière non contrôlée après qu'un cale-bouche soit mis en place, avec notamment un risque d'avaler le cale bouche. De plus, ces cale-bouches n'ont généralement pas de moyen de maintien ou de manipulation et le praticien n'a donc que peu de possibilité d'adapter leur positionnement si le patient ne coopère pas en ouvrant suffisamment la bouche.

Le document CN 201 098 154 Y montre un instrument utilisable comme repousse-langue pour accéder à l'intérieur de la bouche. Il ne propose aucune fonction de calage des mâchoires.

La présente invention a pour but de résoudre les problèmes évoqués ci-dessus, et vise en particulier, pour des personnes présentant une déficience comportementale, à faciliter l'accès à l'intérieur de la cavité buccale et à assurer ensuite le maintien en position ouverte durant l'examen bucco-dentaire, pour faciliter ce dernier et améliorer l'efficacité et la qualité de l'examen et des soins éventuellement requis.

Elle vise ainsi particulièrement à proposer un dispositif d'accessibilité buccale facile d'utilisation, et de manipulation adaptable en permanence en fonction des besoins du praticien et de l'attitude du patient. Elle vise aussi à fournir un dispositif non traumatique pour les structures dentaires ou pour les tissus muqueux.

Avec ces objectifs en vue, l'invention a pour objet un nouveau dispositif d'accessibilité buccale, pour écarter les arcades dentaires et les maintenir en position écartées, conforme à l'objet de la revendication 1.

Le dispositif selon l'invention permet au praticien ou à un assistant de l'utiliser comme outil actif pour écarter les arcades dentaires d'un patient, après avoir inséré la lame à plat entre les arcades dentaires, en exerçant un mouvement de rotation sur le manche pour faire pivoter la lame et ainsi écarter les arcades dentaires selon les besoins, jusqu'à la positionner sensiblement perpendiculairement. La lame peut alors être maintenue dans cette position par le serrage exercé par les arcades dentaires, sans besoin de maintenir un grand effort de rotation.

L'intérêt et l'originalité d'utilisation du dispositif selon l'invention résident dans la possibilité de lever la barrière de protection de fermeture de la bouche par une introduction buccale facilitée et performante. Ceci doit permettre d'accéder à l'intérieur de la cavité buccale et de la maintenir ensuite ouverte durant l'examen bucco-dentaire. Ce dispositif est plus adapté qu'une cale intra-buccale classique plus difficile à mettre en place et à maintenir entre les arcades dentaires durant l'examen.

Des explications complémentaires sur l'utilisation du dispositif seront données par la suite.

On notera cependant dès maintenant l'intérêt de disposer d'une lame d'épaisseur relativement faible, typiquement de l'ordre de quelques millimètres, par exemple entre 3 et 7 mm, et de préférence environ 5 mm, pour permettre une insertion assez facile de la lame entre les arcades dentaires même lorsque le patient ne veut pas ouvrir la bouche.

Les dispositifs connus sont plutôt des dispositifs statiques qui assurent un maintien des arcades dentaires écartées, mais doivent être enlevés, déplacés ou déréglés s'il s'avère nécessaire au cours de l'examen buccal de modifier l'écartement des arcades dentaires ou de modifier les conditions d'accès à la cavité buccale. Pour l'application particulièrement visée par l'invention, c'est-à-dire pour des personnes en situation de handicap comportemental, de tels systèmes présentent une rigidité d'utilisation qui peut être incompatible avec le comportement du patient et traumatisante.

Contrairement à cela, le dispositif selon l'invention, outre qu'il permet au praticien d'écarter les arcades dentaires avec une sensibilité adaptée au comportement du patient, peut aussi être ensuite aisément manipulé toujours en fonction des réactions du patient et des besoins du praticien avec une réactivité beaucoup plus grande. Il s'agit donc là d'un dispositif actif, sur le positionnement duquel le praticien ou un assistant peut intervenir manuellement très rapidement, par comparaison au caractère passif des dispositifs selon l'art antérieur.

Préférentiellement, la lame a une extrémité de forme arrondie, facilitant son insertion entre les arcades dentaires, en évitant les risques de blessures. Dans le même but, les bords de la lame sont aussi arrondis, ce qui facilite aussi le glissement contre les dents ou les gencives lors du pivotement de la lame, et évite les risques de blessures des muqueuses, ou des lèvres, lorsque la manipulation de la spatule peut amener la lame au contact de ces parois.

La partie plate de la lame a typiquement une longueur globale de 60 à 100 mm, par exemple 80 mm, suffisante pour pénétrer la cavité buccale, et sans entraîner un encombrement excessif. La lame peut aussi accessoirement servir d'abaisse-langue. La lame a une largeur diminuant à partir de son extrémité portant le manche vers son extrémité arrondie. Cette variation de largeur le long de la lame, par exemple de 25 à 35 mm, permet donc d'adapter l'écartement souhaité en fonction de la profondeur d'insertion de la lame dans la bouche. En outre, la lame comporte sur ses bords des crans répartis sur sa longueur. Ces crans, par exemple au nombre de trois de chaque côté de la lame, permettent un calage de la lame entre les dents maxillaires et mandibulaires, pour éviter tout glissement de la lame. Le manche s'étend sensiblement parallèlement à la direction longitudinale de la lame et est décalé par rapport au plan moyen de ladite lame. Ce décalage, qui peut être de l'ordre de 15 à 30 mm, par exemple de 25 mm, permet d'accroître le couple de pivotement exercé sur la lame, et donc la force d'écartement des arcades dentaires, lorsque l'utilisateur effectue une rotation du manche. De plus, il permet aussi, dans certaines positions de la lame, d'assurer un écartement du manche par rapport aux joues et donc de faciliter la manipulation.

Pour faciliter sa prise en main, le manche a une forme allongée, par exemple de 80 mm de long, et a section de forme arrondie ou ovale, permettant d'assurer au mieux le couple et les efforts sur la lame. Il pourra aussi comporter une surface antidérapante. La longueur totale de la spatule doit notamment permettre d'assurer un effet de levier pour exercer une force supérieure à la force musculaire de fermeture buccale.

Comme indiqué précédemment, l'épaisseur de la lame sera suffisamment réduite pour faciliter son insertion entre les arcades dentaires bouche fermée. Inversement, cette épaisseur devra être suffisante pour résister en torsion, sans déformation gênante lors de l'utilisation. Cela dépend du matériau utilisé et aussi de la longueur de la lame. Une épaisseur de 5 m environ s'est avérée correcte pour une lame d'environ 125 mm de long, réalisée entièrement en une matière plastique telle que notamment du polyéthylène, permettant d'obtenir une surface lisse facile à nettoyer.

La lame peut aussi être réalisée en un autre matériau résistant, par exemple en métal, revêtu de matière plastique ou caoutchouc.

Préférentiellement, la spatule pourra être réalisée d'une seule pièce, manche et lame, en matière plastique moulée. On pourra utiliser une résine thermoplastique, de type polyéthylène à usage alimentaire, du polypropylène, etc. Le matériau choisi doit assurer une bonne résistance mécanique en flexion et torsion, une résistance aux chocs, à l'usure et aux entailles et éraflures susceptibles d'être provoquées par les dents du patient ou les autres outils du praticien. Il doit aussi résister sans déformation aux températures élevées de stérilisation, typiquement 134°C pendant 18 minutes à une pression de 5 bars.

D'autres caractéristiques et avantages apparaîtront dans la description qui va être faite d'un exemple de spatule conforme à l'invention.

On se reportera aux dessins annexés dans lesquels :

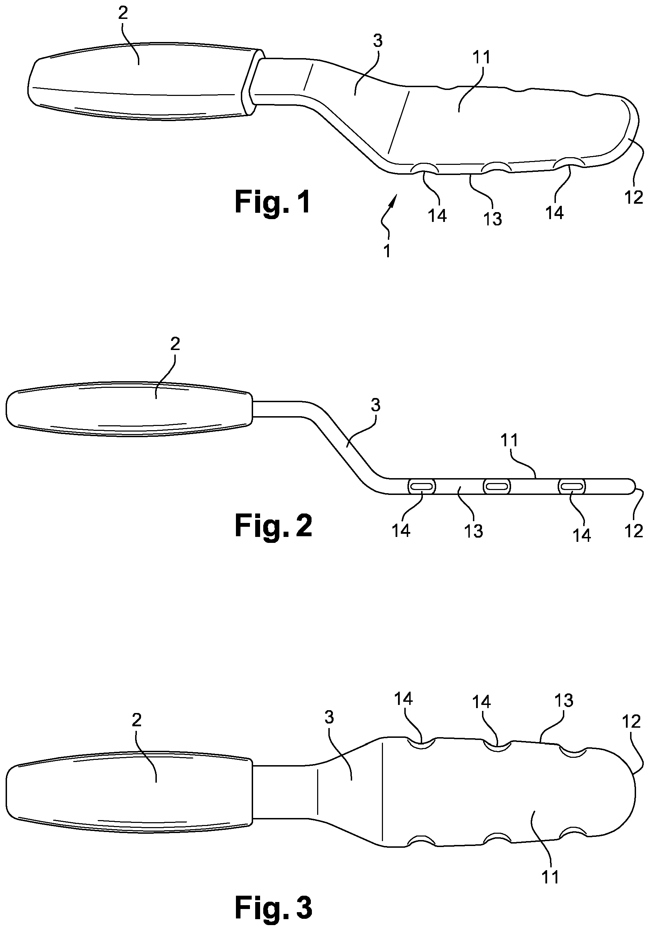

- la figure 1 est une vue en perspective de la spatule,
- la figure 2 est une vue latérale,
- la figure 3 est une vue de dessus,
- les figures 4 à 6 illustrent des exemples d'utilisation de la spatule.

La spatule comporte une lame 1 et un manche de préhension 2, décalés l'un par rapport à l'autre et reliés par une partie inclinée 3. La partie plate 11 de la lame a par exemple une longueur de 80 mm, son extrémité 12 ayant une forme en demi-cercle, de rayon 12,5 mm par exemple. La largeur de la lame varie d'environ 25 mm à proximité de son extrémité arrondie 12, à 35 mm à proximité de la partie inclinée 3 de liaison au manche. Les bords 13 de la lame sont arrondis sur tout son pourtour, et des crans 14 répartis sont formés sur les bords opposés de la lame. L'épaisseur de la lame est typiquement de 5 mm.

Le manche à une longueur d'environ 80 mm et une section de forme ovale ou elliptique, de 26 mm de large pour 18 mm d'épaisseur par exemple.

Dans l'exemple présenté, la spatule est réalisée d'une pièce par moulage, en polyéthylène.

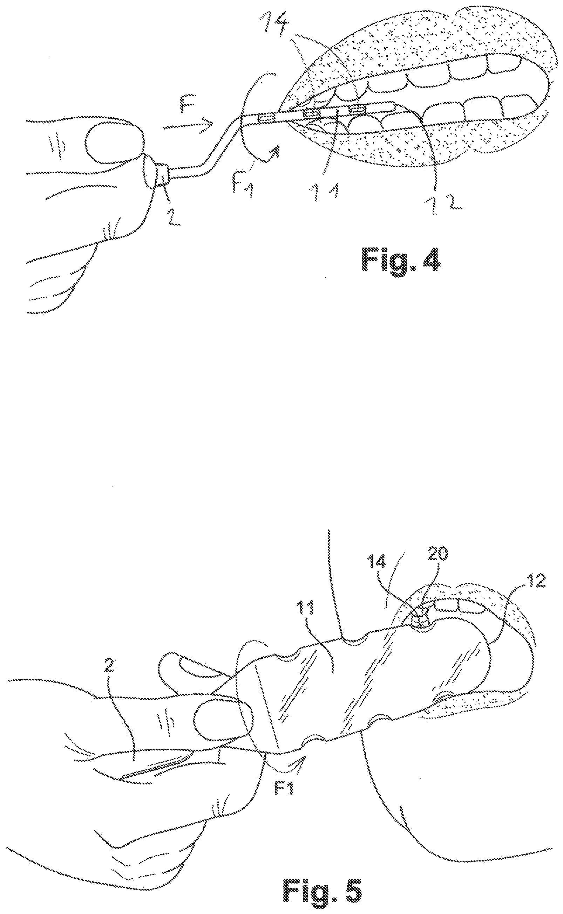

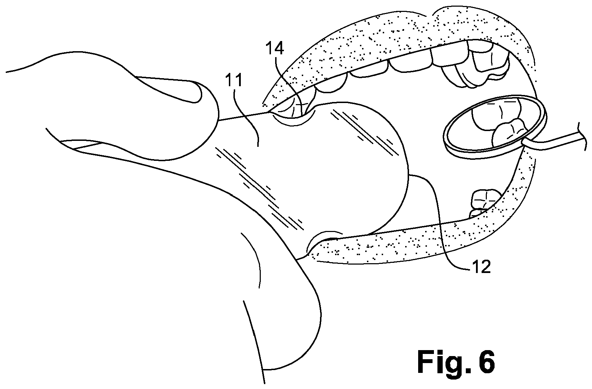

La lame 1 permet par sa forme d'être insérée dans la cavité buccale entre les arcades dentaires 20 pratiquement en contact ou à peine écartées, comme on le voit figure 4. Grâce à sa faible épaisseur, la partie plate 11 de la lame 1 peut être insérée entre les dents selon sa direction longitudinale, selon la flèche F de la figure 4, ou éventuellement latéralement. La forme arrondie de l'extrémité 12, et les arrondis des bords de la lame permettent cette insertion sans risque de blesser les dents ou la cavité buccale. Un mouvement de rotation du manche 2, selon la flèche F1, permet ensuite d'exercer une force suffisante pour permettre l'ouverture buccale, en position telle que représentée figure 5. Les crans ou encoches 14 permettent de caler les dents maxillaires et mandibulaires et créer ainsi un calage des arcades lors de l'examen buccal, symbolisé par l'utilisation du miroir 21 représenté figure 6. La lame peut être plus ou moins avancée dans la cavité buccale pour, grâce à la largeur variable de la lame, adapter l'écartement des arcades dentaires selon les besoins. Une adaptation de l'écartement peut aussi être obtenue par un pivotement de la spatule sur elle-même, l'utilisateur pouvant aussi la maintenir au niveau de la lame, comme représenté figure 6, pour une plus grande précision du positionnement.

Les dimensions, formes et matériaux de l'exemple qui vient d'être décrit sont donnés à titre d'exemple et ne sont nullement limitatifs. On pourra notamment prévoir une gamme de spatules de différentes tailles, pour pouvoir adapter la dimension de la spatule en fonction du patient.

La spatule selon l'invention est notamment destinée à la réalisation d'examens bucco-dentaires de personnes en situation de handicap comportemental, pour les professionnels de santé bucco-dentaire. Les chirurgiens dentistes ou leurs assistants peuvent ainsi manipuler la spatule au cours de l'intervention, en fonction des besoins d'écartement et d'accessibilité à la cavité buccale. L'utilisation de la spatule selon l'invention peut notamment permettre, pour des personnes en situation de handicap comportemental, de limiter les indications de soins sous anesthésie générale.

L'invention peut aussi être avantageusement utilisée par les parents, auxiliaires de vie et éducateurs spécialisés pour maintenir une hygiène bucco-dentaire aux personnes présentant une déficience comportementale.

## Revendications

1. Dispositif d'accessibilité buccale pour écarter les arcades dentaires et les maintenir en position écartées, constitué d'une spatule comportant
- une partie en forme de lame rigide (11), plate et allongée, ayant une extrémité (12) adaptée pour en faciliter l'insertion entre les arcades dentaires d'un patient humain, et une largeur correspondant à un écartement souhaité entre les arcades dentaires du patient, la lame (11) ayant une largeur diminuant du manche vers l'extrémité et comportant sur ses bords des crans (14) répartis sur sa longueur, et
- un manche (2) lié rigidement à une extrémité de la lame et conformé de manière à permettre de faire pivoter la spatule selon un axe longitudinal par un pivotement manuel du manche, le manche (2) s'étendant sensiblement parallèlement à la direction longitudinale de la lame (11) et étant décalé par rapport au plan moyen de ladite lame.

2. Dispositif selon la revendication 1, **caractérisé en ce que** la lame (11) a une épaisseur de 3 à 7 mm.

3. Dispositif selon la revendication 1, **caractérisé en ce que** la lame (11) a une extrémité (12) de forme arrondie.

4. Dispositif selon la revendication 1, **caractérisé en ce que** les bords (13) de la lame sont arrondis.

5. Dispositif selon la revendication 1, **caractérisé en ce que** le manche (2) est allongé et a section de forme arrondie ou ovale.

6. Dispositif selon la revendication 1, **caractérisé en ce qu'**au moins la lame (11) est réalisée entièrement, ou au moins comporte un revêtement, en matière plastique, plus particulièrement en polyéthylène.

7. Dispositif selon la revendication 1, **caractérisé en ce qu'**il est réalisé d'une seule pièce en matière plastique moulée.

## Patentansprüche

1. Vorrichtung zum Zugriff auf den Mundraum, um die Zahnbögen zu beabstanden und sie in beabstandeter Position zu halten, gebildet von einem Spatel, aufweisend
- einen Teil in Form einer starren, flachen und länglichen Klinge (11) mit einem Ende (12), das ausgebildet ist, um dessen Einführen zwischen die Zahnbögen eines Patienten zu erleichtern, und in einer Breite, die der gewünschten Beabstandung zwischen den Zahnbögen des menschlichen Patienten entspricht, wobei die Klinge (11) eine Breite hat, die vom Griff zum Ende abnimmt und auf ihren Rändern über ihre Länge verteilte Rasten (14) aufweist, und
- einen Griff (2), der starr mit einem Ende der Klinge verbunden und derart ausgebildet ist, dass er erlaubt, den Spatel gemäß einer Längsachse durch manuelles Drehen des Griffs zu drehen, wobei sich der Griff (2) etwa parallel zur Längsrichtung der Klinge (11) erstreckt und in Bezug zur mittleren Ebene der Klinge versetzt ist.

2. Vorrichtung nach Anspruch 1, **dadurch gekennzeichnet, dass** die Klinge (11) eine Dicke von 3 bis 7 mm hat.

3. Vorrichtung nach Anspruch 1, **dadurch gekennzeichnet, dass** die Klinge (11) ein abgerundetes Ende (12) hat.

4. Vorrichtung nach Anspruch 1, **dadurch gekennzeichnet, dass** die Ränder (13) der Klinge abgerundet sind.

5. Vorrichtung nach Anspruch 1, **dadurch gekennzeichnet, dass** der Griff (2) länglich und mit abgerundetem oder ovalem Querschnitt ist.

6. Vorrichtung nach Anspruch 1, **dadurch gekennzeichnet, dass** mindestens die Klinge (11) vollständig aus Kunststoff gefertigt ist oder mindestens eine Beschichtung daraus aufweist, insbesondere aus Polyethylen.

7. Vorrichtung nach Anspruch 1, **dadurch gekennzeichnet, dass** sie in einem einzigen Stück aus geformtem Kunststoff hergestellt ist.

## Claims

1. Oral accessibility device for separating the dental arches and holding the latter in separated position consisting of a spatula comprising:
- a portion forming a planar and elongated rigid blade (11), having one end (12) suitable for facilitating insertion between the dental arches of a human patient, and a width corresponding to a desired separation between the dental arches of the patient, the blade (11) having a length diminishing from the handle towards the end and comprising notches (14) on its edges distributed over its length, and
- a handle (2) rigidly connected to one end of the blade and formed to allow the spatula to be pivoted along a longitudinal axis by a manual pivoting of the handle, the handle (2) extending substantially parallel to the longitudinal direction of the blade (11) and being offset in relation to the mean plane of said blade.

2. Device according to claim 1, **characterised in that** the blade (11) has a thickness from 3 to 7 mm.

3. Device according to claim 1, **characterised in that** the blade (11) has one end (12) of rounded shape.

4. Device according to claim 1, **characterised in that** the edges (13) of the blade are rounded.

5. Device according to claim 1, **characterised in that** the handle (2) is elongated and has a section of rounded or oval shape.

6. Device according to claim 1, **characterised in that** at least the blade (11) is made completely, or at least comprises a coating, of plastic material, more especially polyethylene.

7. Device according to claim 1, **characterised in that** it is made in one part from moulded plastic material.
